# FASCICULE DE BREVET EUROPEEN

(11) **EP 3 302 406 B1**
(45) Date de publication et mention de la délivrance du brevet: **07.10.2020**
(21) Numéro de dépôt: 16741051.3
(22) Date de dépôt: 02.06.2016
(51) Int. Cl.: A61K 8/25, A61Q 3/02, A61K 8/87, A61K 8/02, A61K 8/04

(54) **COMPOSITION COSMÉTIQUE DE VERNIS À ONGLE AQUEUX PELLICULABLE À BASE DE POLYURÉTHANE STABILISÉ**
ABZIEHBARE WÄSSRIGE KOSMETISCHE NAGELLACKZUSAMMENSETZUNG MIT STABILISIERTEM POLYURETHAN
STRIPPABLE AQUEOUS COSMETIC NAIL VARNISH COMPOSITION CONTAINING STABILISED POLYURETHANE

(30) Priorité: 03.06.2015 FR 1555058
(43) Date de publication de la demande: 11.04.2018
(73) Titulaire: Fiabila, 28130 Maintenon (FR)
(72) Inventeur: MAGNET, Serge, 78530 Buc (FR)
(74) Mandataire: Ipsilon
(86) Numéro de dépôt international: PCT/FR2016/051317
(87) Numéro de publication internationale: WO 2016/193626

(56) Documents cités:
- EP-A1- 0 745 372
- WO-A1-00/27347
- WO-A1-2013/144871
- WO-A1-2015/036410
- FR-A1- 2 791 557
- AEROSIL: "Versatile and Effective", DEGUSSA, TECHNICAL INFORMATION,, no. TI 1251, 1 mars 2003 (2003-03-01), pages 1-21, XP003026229,

## Description

La présente invention concerne les vernis à ongles en phase aqueuse, incolores ou colorés, et plus particulièrement les vernis se démaquillant par pelliculage.

### Etat de l'art :

Le marché des vernis à ongle est aujourd'hui dominé par les compositions de vernis à ongles préparées en phase solvant et sont à base de nitrocellulose combinée à une autre résine et à des plastifiants en solution dans des solvants volatils. Des considérations écologiques liées à l'évaporation de ces solvants dans l'atmosphère lors de l'application de ces vernis ainsi que les risques potentiels liés à l'inflammabilité de ces solvants ont conduit les fabricants à développer des vernis à ongles en phase aqueuse.

Cependant les vernis à ongle à l'eau montrent une durabilité sur l'ongle inférieure à celle des vernis en phase solvant organique et sont plus difficiles à démaquiller. A cet effet, ils nécessitent néanmoins les mêmes dissolvants organiques que les vernis à ongles en phase solvant non-aqueux.

Ont alors été développés des vernis à ongles en phase aqueuse pouvant se démaquiller par simple pelage (ou pelliculage), opération consistant à enlever de l'ongle le film déposé en l'entamant sur un coin de l'ongle, à le soulever et le tirer par cette extrémité pour le décoller de l'ongle, idéalement d'une seule pièce. De tels vernis aqueux pelables ne nécessitent pas l'usage d'un dissolvant pour leur démaquillage et représentent ainsi la solution technique la plus écologique.

Ces vernis aqueux pelables (ou pelliculables) renferment généralement en tant qu'agent filmogène une dispersion aqueuse de polyuréthane. De nombreux brevets mentionnent de telles dispersions.

Ainsi le brevet EP0143480 A2 décrit un vernis aqueux pelliculable préparé à partir de dispersions aqueuses de polyuréthane formé de polyéthers ou polyesters avec des isocyanates aliphatiques ou aromatiques.
Le brevet EP391322 B1 décrit l'utilisation d'une dispersion aqueuse d'un liant polyuréthane et/ou d'un copolymère polyuréthane/acrylate dans la composition de vernis à ongle, en présence d'un épaississant acrylique.

De même, le brevet FR2711059 revendique l'utilisation de dispersion aqueuse de polyester-polyuréthane anionique renfermant des particules de taille comprise entre 2 et 40 nm et donnant un film d'une dureté améliorée.

Le brevet EP0423471 décrit une composition de vernis à ongle à base de dispersion aqueuse de polyuréthane-polyurée. Pour améliorer la tenue sur l'ongle, le brevet FR2718350 propose la combinaison d'une dispersion aqueuse de particules de polyuréthane et de polymères radicalaires à groupement carboxylique dans des proportions qui dépendent des températures de transition vitreuse respectives des polymères.

Des combinaisons de différentes dispersions de polyuréthane ayant des élongations à la rupture différentes ont également été décrites dans le brevet US5830443 pour former un vernis dur, mais flexible. Enfin le brevet FR2831797 décrit un vernis à ongle aqueux à base d'une dispersion aqueuse de polyuréthane spécifique, obtenu par polycondensation du diisocyanate de tétraméthyl xylylène avec un diol.

Ces brevets décrivent également l'utilisation des additifs et autres ingrédients entrant dans la composition des vernis à ongles en phase aqueuse et connus de l'homme de l'art.

Cependant, des limitations techniques ont été observées en ce qui concerne la préparation et l'utilisation de ces vernis à ongles à base de dispersion de polyuréthane, notamment des difficultés à stabiliser les pigments minéraux denses tels que les oxydes métalliques et les charges apportant des effets particuliers telles que les nacres. En effet ces pigments et charges ont tendance à sédimenter dans le temps malgré l'ajout d'additifs pour contrôler la rhéologie des vernis. Ce phénomène est d'autant plus rapide que la température est élevée.

On connaît également de WO 00/27347 et FR 2791557 des compositions de vernis à ongles aqueux contenant une dispersion de polyuréthane et du silicate de magnésium et sodium, en tant qu'ajout anti-décantation des pigments.
Il est aussi connu de EP 0745372, dans son exemple 4, un vernis à ongles renfermant 38 % de dispersion aqueuse de polymère acrylique, 50 % de dispersion de polyuréthane, 0,7 % de silice colloïdale et des colorants.

Les épaississants acryliques et polyuréthanes associatifs généralement utilisés pour ajuster le profil rhéologique des préparations aqueuses donnent très souvent une structure inappropriée au vernis qui se traduit par un tendu médiocre à l'application ainsi que des séparations de phase, appelées synérèse, lors des tests de stabilité. L'utilisation d'agents rhéologiques à base de cellulose ou de gommes naturelles provoque généralement une perte de transparence de la préparation à base de dispersion de polyuréthane qui devient blanche ; ceci se traduit lors de la coloration du vernis par une différence de couleur entre le vernis liquide et le film sec appliqué sur l'ongle. Les agents minéraux anti-sédimentation tels que les bentonites et les silicates d'aluminium montrent une compatibilité limitée avec les dispersions de polyuréthane et leur dispersion dans le milieu est difficile. Ils affectent également la brillance du film de vernis.

### Buts de l'invention :

Un premier but de la présente invention est de pallier les inconvénients des compositions de vernis pelables de l'art antérieur et de proposer une composition de vernis à ongle en phase aqueuse incolore ou colorée préparée avec une dispersion aqueuse de polyuréthane en tant qu'agent filmogène, qui présente une stabilité au stockage améliorée, tout en conservant les propriétés d'application et l'effet esthétique attendus et pouvant se démaquiller par simple pelliculage.

Un autre but de l'invention est de proposer une composition de vernis à ongle en phase aqueuse préparée avec une dispersion aqueuse de polyuréthane en tant qu'agent filmogène, qui donne après application, un film brillant, suffisamment durable et résistant à l'eau et démaquillable facilement par simple pelliculage.

### Description de l'invention :

A cet effet, la présente invention propose une composition de vernis à ongle aqueux pelliculable, renfermant en tant qu'agent filmogène une dispersion aqueuse de polyuréthane, caractérisée en ce qu'elle comprend également de 2 à 5 % en poids d'une dispersion aqueuse de silice colloïdale anionique contenant des contre-ions sodium ou ammonium et entre 30 et 40 % en poids de particules de silice.

Les inventeurs ont en effet découvert que l'utilisation de dispersion aqueuse de silice colloïdale comme agent anti-sédimentation permet, du fait des interactions entre les groupes chimiques présents à la surface des particules de silice dispersées et la surface des particules de polyuréthane, et la surface des pigments et des charges éventuellement présents, d'apporter une stabilité améliorée à l'ensemble de la composition de vernis. Une telle stabilité est supérieure à celle apportée par les additifs rhéologiques utilisés dans l'art antérieur pour de telles compositions de vernis aqueux pelliculables.

L'avantage de la présente invention est donc l'obtention d'un vernis à ongle ne présentant pas de sédimentation, notamment des pigments et charges dans les flacons tout en conduisant, après application, à un film brillant, suffisamment durable et résistant à l'eau. Cette composition selon l'invention est apte à former sur l'ongle un film qui, après séchage, peut être facilement démaquillé par simple pelliculage.

De manière avantageuse, la taille des particules de la silice colloïdale est comprise entre 5 et 45 nm, de préférence entre 5 et 30 nm, et de préférence encore entre 7 et 25 nm. La distribution des tailles de ces particules, qui sont donc des nano-particules, peut être une distribution mono ou poly-dispersée.

Les effets anti-sédimentation de la dispersion de silice colloïdale se sont avérés particulièrement intéressants lorsque la composition de vernis à ongle aqueux comprend une proportion de dispersion de silice colloïdale comprise entre 2 et 5% en poids par rapport au poids total de la composition. La quantité minimale de silice colloïdale à ajouter dépend en particulier de la nature des pigments et des charges introduites dans la formulation de vernis. Par exemple, les particules de nacres étant plus grosses et plus lourdes que les particules de pigments organiques, elles nécessitent une concentration en silice colloïdale plus élevée.

La présente invention s'applique en particulier à des compositions de vernis à ongle aqueux dans lesquelles la dispersion de polyuréthane est une dispersion anionique, à base de polyacrylate, de polyéther, de polyester ou de polycarbonate et d'isocyanate aliphatique et/ou aromatique, ou un mélange de ceux-ci.

L'extrait sec de la dispersion de polyuréthane peut être compris entre 20 et 50%, de préférence entre 20 et 40 % en poids. Des exemples non limitatifs de dispersions de polyuréthane sont notamment les dispersions Alberdingk U6800 ou Alberdingk U5200 commercialisées par la société ALberdingk-Boley , ou encore la dispersion Baycusan C1004 commercialisée par la société Bayer.

La dispersion de polyuréthane renferme avantageusement des particules de taille inférieure à 200 nm, et de préférence inférieure à 100 nm, de façon à préparer des compositions pour vernis à ongles transparentes ou translucides avant l'introduction des colorants ou des pigments.

De manière avantageuse, la dispersion de polyuréthane présente une température de formation de film inférieure ou égale à 30°C et de préférence inférieure à 20°C afin d'assurer de bonnes propriétés filmogènes et une élongation à la rupture de 100% ou plus.

La préparation de la composition pour vernis à ongle selon la présente invention s'effectue par simple mélange des différents constituants de la composition avec la dispersion de polyuréthane, puis l'ajout et le mélange de la dispersion aqueuse de silice colloïdale, en utilisant par exemple un disperseur.

La composition selon la présente invention peut contenir entre 80 % et 98 %, de préférence entre 85 et 97% de dispersion aqueuse de polyuréthane.

La composition selon la présente invention peut renfermer un ou plusieurs pigments et/ou charges organiques ou inorganiques (par exemple des nacres) dans une proportion inférieure ou égale à 10%, de préférence inférieure ou égale à 5% en poids par rapport au poids total de la composition.

La composition de vernis selon la présente invention peut également contenir les additifs et ingrédients communément utilisés pour la préparation de vernis à ongle en phase aqueuse. En particulier elle peut contenir au moins un additif choisi parmi : des co-solvants, des coalescents, des agents anti-mousse, des agents de surface, des agents mouillants, des agents dispersants, des cires, des silicones, des accélérateurs de séchage, des agents de réticulation, des promoteurs d'adhésion, des filtres UV, des agents rhéologiques tels que des épaississants acryliques et polyuréthanes associatifs, des conservateurs, tels que des agents antibactériens et antifongiques nécessaires à la préservation du milieu aqueux, ou un mélange de ceux-ci.

La composition selon la présente invention peut être colorée en utilisant les colorants solubles dans l'eau ou les pigments organiques et inorganiques et laques autorisés par la législation cosmétique.

La composition selon la présente invention peut aussi contenir un ou plusieurs agent(s) actif(s) pour l'entretien et l'amélioration de l'aspect de l'ongle, de préférence à effet non thérapeutique, comme par exemple des agents améliorant le lissage de l'ongle ou apportant une action d'hydratation.

La composition de vernis à ongles selon l'invention peut aussi renfermer, en tant qu'agent filmogène complémentaire (appelé aussi co-liant), de préférence jusqu'à 30% en poids d'une dispersion aqueuse de polymère acrylique ou styrène acrylique dont les particules présentent une taille inférieure à 150 nm, et de préférence inférieure à 100 nm, afin de ne pas trop opacifier la composition. La teneur en copolymère acrylique ou styrène acrylique d'une telle dispersion est de préférence comprise entre 30 et 50%. Si la température de formation de film de la dispersion acrylique ou styrène acrylique est supérieure à 20°C, celle-ci est abaissée à une valeur inférieure ou égale à 25°C par ajout d'agent de coalescence dans la composition selon une technique très largement connue de l'homme de l'art. Un exemple non limitatif d'une telle dispersion acrylique est le Neocryl A1131 commercialisé par la société DSM.

La composition selon l'invention est ainsi apte à former sur l'ongle un film qui, après séchage, peut être démaquillé par simple pelliculage.

La présente invention concerne également un procédé de maquillage et démaquillage d'ongles caractérisé en ce qu'il comprend les étapes suivantes :
i) application sur l'ongle d'une ou plusieurs couches d'une composition de vernis telle que décrite ci-dessus, sous forme d'un film continu
ii) séchage de chacune des couches de vernis à température ambiante
iii) démaquillage du vernis par pelage du film déposé sur l'ongle.

Par température ambiante, on entend une température comprise entre 15 et 25°C environ.

La présente invention va maintenant être décrite plus en détail et illustrée par les exemples, non limitatifs, ci-après :

### Exemples

Dans l'ensemble du texte, sauf indication contraire, les pourcentages sont des pourcentages en poids.

Différentes compositions de vernis à ongles pelables ont été formulées et testées.

La formule générale de la composition de vernis à ongles, appelée composition de base, incolore, comprend les constituants suivants :

| | |
|---|---|
| Dispersion aqueuse de polyuréthane (par ex Alberdingk U5200) | 81 à 96 |
| Dispersion aqueuse d'agent anti sédimentation de 0 (exemples comparatifs) à | 15 |
| Cosolvant (Dowanol DPM) : dérivé de propylène glycol | 2 |
| Bactéricide (Euxyl 90/10) | 1 |
| Epaississant associatif de type HEUR | 0,5 |
| Antimousse | 0,25 |
| Agent de surface | 0,25 |
| | ---- |
| | 100 |

Les tests suivants ont été réalisés sur les différentes compositions de vernis :
- Brillance : Sur une carte Leneta, on applique 100µm de la composition de vernis. Après séchage à 20°C du film obtenu, on mesure sa brillance sous un angle de 60° à l'aide d'un glossmètre Byk Gardner.
   La brillance du vernis coloré est mesurée sur le film obtenu à partir du vernis incolore dans lequel a été ajouté 3% de dispersion pigmentaire contenant 30% d'oxyde de titane.
- Stabilité à 45°C : Un flacon est rempli de composition de vernis à ongles jusqu'au trois quart de sa hauteur puis bouché et placé dans une étuve à 45°C pendant un mois. Aucun phénomène de sédimentation, de séparation de phase, de démixtion de couleur ou de prise en masse ne doit être observé.
- Stabilité à 20°C : Un flacon est rempli de composition de vernis à ongles jusqu'au trois quart de sa hauteur puis bouché et stocké à 20°C pendant plusieurs mois. Aucun phénomène de sédimentation, de séparation de phase, de démixtion de couleur ou de prise en masse ne doit être observé.
- Extrait sec : Dans une coupelle, on pèse entre 0,5 g et 1 g de composition de vernis. On place ensuite cette plaque à l'étuve dans un dessiccateur électrique à 140°C couplé à une balance jusqu'à stabilité complète du poids.
- Aptitude au pelage : La composition de vernis est appliquée sur l'ongle puis séchée à température ambiante. Le film obtenu est ensuite enlevé de l'ongle par pelliculage en l'entamant sur un coin de l'ongle et en le tirant. Le test est positif si le film de vernis s'enlève d'un seul tenant sans se casser et sans laisser de morceaux sur l'ongle.
- Aspect du film : Une composition de vernis est appliquée sous la forme d'un film de 100 micromètres humide sur une plaque de verre à l'aide d'un applicateur puis séchée à 20°C. L'absence de fissure, l'absence de grains et la transparence du film sont observées.

Les exemples 1 à 16 suivants présentent les différentes compositions formulées et testées.

### Exemple comparatif 1 (référence) :

Vernis incolore selon la formule générale ne contenant pas d'agent anti sédimentation

### Exemple 2 :

Vernis incolore selon la présente invente préparé selon la formule générale préparé avec 5% de dispersion de silice colloïdale de taille de particule de 22 nm dans l'eau par exemple le Ludox AS40 commercialisé par Grace ou le Bindzil 40NH3130 commercialisés par AKZO Nobel.

### Exemple 3 (comparatif) :

Vernis incolore selon la formule générale préparé avec 5% de dispersion à 10% dans l'eau de silicate synthétique (Laponite XLS commercialisé par Rockwood).

### Exemple 4 (comparatif) :

Vernis incolore selon la formule générale préparé avec 15% d'un gel à 5% dans l'eau de bentonite (Optigel CK commercialisé par Rockwood).

### Exemple 5 (comparatif) :

Vernis incolore selon la formule générale préparé avec 15 % d'un gel à 5% d'épaississant associatif type ASE dans l'eau : l'épaississant acrylique Acrysol ASE60 commercialisé par DOW Chemicals

### Exemple 6 (comparatif) :

Vernis incolore selon la formule générale préparé avec 12 % d'un gel d'hydroxycellulose à 5% dans l'eau (Natrosol 250HR commercialisé par Ashland).

### Résultats :

Les caractéristiques des vernis incolores obtenus dans les exemples 1 à 6 sont présentées dans le tableau 1 ci-après :

**Tableau 1**

| | **Ex 1 (Réf.)** | **Ex 2** | **Ex 3 (comp.)** | **Ex 4 (comp.)** | **Ex 5 (comp.)** | **Ex 6 (comp.)** |
|---|---|---|---|---|---|---|
| **Agent anti-sédimentation** | / | Silice colloïdale | Silicate | Bentonite | Epaississant acrylique | Gel d'hydro-cellulose |
| **Aspect de la base** | translucide | translucide | translucide | trouble | opaque | opaque |
| **Aspect du film** | Transparent | Transparent | Transparent | Trouble | trouble | opaque |
| **Extrait sec (%)** | 38 | 38,1 | 36,4 | 36,4 | 36,4 | 36,4 |
| **Brillance (60°, UB)** | 94 | 95 | 85 | 70 | 70 | 70 |
| **Pelage** | OK | OK | OK | OK | OK | OK |

Aspect de la base : concerne l'aspect visuel de la composition préparée, avant application (dans le flacon) ;
Aspect du film : concerne l'aspect visuel du vernis après application sur plaque de verre.

Il apparaît que la dispersion de silice colloïdale (exemple 2) présente la meilleure compatibilité avec la dispersion aqueuse de polyuréthane ce qui est mis en évidence par la transparence et la brillance du film de vernis obtenu.

### Exemple 7 (comparatif) :

Vernis incolore, selon la présente invention, préparé selon la formule générale avec 1% de dispersion de silice colloïdale de taille de particule de 22 nm dans l'eau neutralisée par l'ammoniaque par exemple le Ludox AS40 commercialisé par Grace. Des résultats identiques ont été obtenus avec le Bindzil 40NH3130 commercialisé par AKZO Nobel.

### Exemple 8 :

Vernis incolore, selon la présente invention, préparé selon la formule générale avec 2% de dispersion de silice colloïdale de taille de particule de 22 nm dans l'eau neutralisée par l'ammoniaque par exemple le Ludox AS40 commercialisé par Grace. Des résultats identiques ont été obtenus avec le Bindzil 40NH3130 commercialisé par AKZO Nobel.

### Exemple 9 (comparatif) :

Vernis incolore, selon la présente invention, préparé selon la formule générale avec 1% de dispersion de silice colloïdale de taille de particule de 17 nm à 30% dans l'eau neutralisée par l'ammoniaque : le Levasil 200N30 commercialisé par AKZO Nobel.

### Exemple 10 :

Vernis incolore, selon la présente invention, préparé selon la formule générale avec 2% de dispersion de silice colloïdale de taille de particule de 17 nm à 30% dans l'eau neutralisée par l'ammoniaque : le Levasil 200N30 commercialisé par AKZO Nobel.

### Exemple 11 :

Vernis incolore, selon la présente invention, préparé selon la formule générale avec 5% de dispersion de silice colloïdale de taille de particule de 17 nm à 30% dans l'eau neutralisée par l'ammoniaque : le Levasil 200N30 commercialisé par AKZO Nobel.

### Exemple 12 (comparatif) :

Vernis incolore, selon la présente invention, préparé selon la formule générale avec 1% de dispersion de silice colloïdale de taille de particule de 7 nm à 30% dans l'eau neutralisée par l'ammoniaque : le Ludox AS30 commercialisé par Grace.

### Exemple 13 :

Vernis incolore, selon la présente invention, préparé selon la formule générale avec 3% de dispersion de silice colloïdale de taille de particule de 7 nm à 30% dans l'eau neutralisée par l'ammoniaque : le Ludox AS30 commercialisé par Grace.

### Exemple 14 :

Vernis incolore, selon la présente invention, préparé selon la formule générale avec 5% de dispersion de silice colloïdale de taille de particule de 22 nm à 40% dans l'eau neutralisée par la soude : le Ludox HS40 commercialisé par Grace.

### Exemple 15 :

Vernis incolore, selon la présente invention, préparé selon la formule générale avec 5% de dispersion de silice colloïdale de taille de particule de 12 nm à 40% dans l'eau neutralisée par la soude : le Ludox TM40 commercialisé par Grace.

### Exemple 16 :

Vernis incolore, selon la présente invention, préparé selon la formule générale avec 5% de dispersion de silice colloïdale non ionique de taille de particule de 22 nm à 40% dans l'eau : le Ludox TMA commercialisé par Grace.

### Exemple 17 :

Des vernis colorés sont préparés à partir des vernis incolores des exemples 1 à 16 et leur stabilité à 20°C et à 45°C est observée. Les différentes formules testées sont les suivantes :
- Formule de test 1 :
   Vernis coloré obtenu par ajout de 3% de dispersion à 30% de pigment organique jaune 1 dans le vernis incolore des exemples 1 à 3.
- Formule de test 2 :
   Vernis coloré obtenu par ajout de 1% de dispersion à 30% de pigment organique rouge 30 et de 2% de nacre de granulométrie comprise entre 20 et 160 micromètres dans le vernis incolore des exemples 1 à 15.
- Formule de test 3 :
   Vernis coloré obtenu par ajout de 3% de dispersion à 30% d'oxyde de titane dans le vernis incolore des exemples 1 à 15.
- Formule de test 4 :
   Vernis coloré obtenu par ajout de 3% de dispersion à 30% d'oxyde de fer jaune dans le vernis incolore des exemples 1 à 3.
- Formule de test 5 :
   Vernis coloré obtenu par ajout de 3% de dispersion à 30% d'oxyde de fer rouge dans le vernis incolore des exemples 1 à 3.
- Formule de test 6 :
   Vernis coloré obtenu par ajout de 3% de dispersion à 30% de bleu ultramarine dans le vernis incolore des exemples 1 à 3.
- Formule de test 7 :
   Vernis coloré obtenu par ajout de 10% de paillettes silver 0.008 (environ 200 µm) dans le vernis incolore des exemples 1 à 6.

### Résultats de stabilité au stockage :

Les résultats de stabilité après 6 mois à 20°C sont présentés dans le tableau 2 ci-après :

**Tableau 2**

| | **Exemple 1 (comp.)** | **Exemple 2 Selon l'invention** | **Exemple 3 (comp.)** |
|---|---|---|---|
| **Formule test 1** | stable | stable | stable |
| **Formule test 2** | sédimentation des nacres | Stable | sédimentation des nacres |
| **Formule test 3** | stable | stable | stable |
| **Formule test 4** | légère sédimentation | stable | stable |
| **Formule test 5** | légère sédimentation | stable | très légère sédimentation |
| **Formule test 6** | légère sédimentation | stable | stable |
| **Formule test 7** | sédimentation des paillettes | stable | sédimentation des paillettes |

Les résultats de stabilité après 1 mois à 45°C sont regroupés dans le tableau 3 ci-après :

**Tableau 3**

| | **Exemple 1 (comp.)** | **Exemple 2 Selon l'invention** | **Exemple 3 (comp.)** |
|---|---|---|---|
| **Formule test 1** | stable | stable | stable |
| **Formule test 2** | sédimentation des nacres | Stable | sédimentation des nacres |
| **Formule test 3** | légère sédimentation | stable | très légère sédimentation |
| **Formule test 4** | sédimentation | stable | sédimentation |
| **Formule test 5** | sédimentation | stable | sédimentation |
| **Formule test 6** | sédimentation | stable | sédimentation |
| **Formule test 7** | sédimentation des paillettes | stable | sédimentation des paillettes |

Ces résultats montrent que la meilleure stabilité est obtenue avec la composition de vernis selon la présente invention contenant la dispersion de silice colloïdale.

**Stabilité 1 mois à 45°C** : les effets de la concentration en dispersion de silice colloïdale et de la taille des particules de silice ont été testés. Les résultats sont regroupés dans le tableau 4.

**Tableau 4**

| **exemple** | **7** | **8** | **2** | **9** | **10** | **11** | **12** | **13** |
|---|---|---|---|---|---|---|---|---|
| % dispersion de silice | 1 | 2 | 5 | 1 | 2 | 5 | 1 | 3 |
| Taille de particule (nm) | 22 | 22 | 22 | 17 | 17 | 17 | 7 | 7 |
| Contre-ion | NH4⁺ | NH4⁺ | NH4⁺ | NH4⁺ | NH4⁺ | NH4⁺ | NH4⁺ | NH4⁺ |
| Formule test 1 | sédimen tation | stable | stable | sédimen tation | stable | stable | sédimen tation | stable |
| Formule test 2 | sédimen tation | stable | stable | sédimen tation | stable | stable | sédimen tation | stable |
| Formule test 4 | sédimen tation | stable | stable | sédimen tation | stable | stable | sédimen tation | stable |

Ces résultats confirment que la quantité minimale de dispersion de silice colloïdale doit être supérieure à 1% en poids dans la composition de vernis.

**Stabilité à 45°C :** l'influence du type de stabilisation des particules de silice a été observée, et notée dans le tableau 5 ci-après.

**Tableau 5**

| **exemple** | **2** | **14** | **15** | **16** |
|---|---|---|---|---|
| % dispersion de silice | 5 | 5 | 5 | 5 |
| Taille de particule nm | 22 | 22 | 12 | 22 |
| Contre-ion | NH4⁺ | Na⁺ | Na⁺ | aucun |
| Formule test 1 | stable | stable | stable | sédimentation |
| Formule test 2 | stable | stable | stable | sédimentation |

Il apparaît qu'une dispersion de silice colloïdale, stabilisée de façon ionique, de préférence avec des ions sodium ou ammonium, permet d'apporter une meilleure stabilité à la composition de vernis à base de polyuréthane.

## Revendications

1. Composition de vernis à ongle aqueux pelliculable, renfermant en tant qu'agent filmogène une dispersion aqueuse de polyuréthane, **caractérisée en ce qu'**elle comprend également de 2 à 5 % en poids d'une dispersion aqueuse de silice colloïdale anionique contenant des contre-ions sodium ou ammonium et entre 30 et 40 % en poids de particules de silice.

2. Composition selon la revendication 1, **caractérisée en ce que** la dispersion aqueuse de silice colloïdale renferme des particules de silice colloïdale de taille comprise entre 5 et 45 nm.

3. Composition selon l'une des revendications 1 ou 2, **caractérisée en ce que** la taille des particules de la silice colloïdale est comprise entre 5 et 30 nm, de préférence encore entre 7 et 25 nm.

4. Composition selon l'une quelconque des revendications précédentes, **caractérisée en ce que** la dispersion de polyuréthane est une dispersion anionique, à base de polyacrylate, de polyéther, de polyester ou de polycarbonate et d'isocyanate aliphatique et/ou aromatique, ou un mélange de ceux-ci.

5. Composition selon l'une quelconque des revendications précédentes, **caractérisée en ce que** la dispersion de polyuréthane renferme des particules de taille inférieure à 200 nm, de préférence inférieure à 100 nm et présente une valeur d'extrait sec comprise entre 20% et 50% en poids, de préférence entre 20 et 40 % en poids.

6. Composition selon l'une quelconque des revendications précédentes, **caractérisée en ce que** la dispersion de polyuréthane présente une température de formation de film inférieure ou égale à 30°C, de préférence inférieure à 20°C.

7. Composition selon l'une quelconque des revendications précédentes, **caractérisée en ce qu'**elle renferme, de préférence jusqu'à 30% en poids d'une dispersion aqueuse de polymère acrylique ou styrène acrylique dont les particules présentent une taille inférieure à 150 nm et de préférence inférieure à 100 nm, en tant qu'agent filmogène complémentaire.

8. Composition selon l'une quelconque des revendications précédentes, **caractérisée en ce qu'**elle renferme un ou plusieurs pigments et/ou charges organiques ou inorganiques dans une proportion inférieure ou égale à 10%, de préférence inférieure ou égale à 5% en poids par rapport au poids total de la composition.

9. Composition selon l'une quelconque des revendications précédentes, **caractérisée en ce qu'**elle contient également au moins un additif choisi parmi : des co-solvants, des coalescents, des agents anti-mousse, des agents de surface, des agents mouillants, des agents dispersants, des cires, des silicones, des accélérateurs de séchage, des agents de réticulation, des promoteurs d'adhésion, des filtres UV, des agents rhéologiques tels que des épaississants acryliques et polyuréthanes associatifs, des conservateurs, tels que des agents antibactériens et antifongiques nécessaires à la préservation du milieu aqueux, ou un mélange de ceux-ci.

10. Composition selon l'une quelconque des revendications précédentes, **caractérisée en ce qu'**elle contient un ou plusieurs agent(s) actif(s) pour l'entretien et la beauté des ongles, de préférence à effet non thérapeutique.

11. Composition selon l'une quelconque des revendications précédentes, **caractérisée en ce qu'**elle est apte à former sur l'ongle un film qui, après séchage, peut être démaquillé par simple pelliculage.

12. Procédé de maquillage et démaquillage d'ongles **caractérisé en ce qu'**il comprend les étapes suivantes :
i) application sur l'ongle d'une ou plusieurs couches d'une composition de vernis selon l'une quelconque des revendications 1 à 11, sous forme d'un film continu
ii) séchage de chacune des couches de vernis à température ambiante
iii) démaquillage du vernis par pelage du film déposé sur l'ongle.

## Patentansprüche

1. Abziehbare wässrige Nagellackzusammensetzung, die als Filmbildner eine wässrige Polyurethandispersion einschließt, **dadurch gekennzeichnet, dass** sie ebenfalls 2 bis 5 Gew.-% einer wässrigen Dispersion anionischen kolloidalen Siliziumdioxids umfasst, enthaltend Natrium- oder Ammonium-Gegenionen, und zwischen 30 und 40 Gew.-% Siliziumdioxidpartikel.

2. Zusammensetzung nach Anspruch 1, **dadurch gekennzeichnet, dass** die wässrige Dispersion kolloidalen Siliziumdioxids Partikel kolloidalen Siliziumdioxids in einer Größe zwischen 5 und 45 nm einschließt.

3. Zusammensetzung nach einem der Ansprüche 1 oder 2, **dadurch gekennzeichnet, dass** die Größe der Partikel des kolloidalen Siliziumdioxids zwischen 5 und 30 nm, besonders bevorzugt zwischen 7 und 25 nm liegt.

4. Zusammensetzung nach einem der vorangehenden Ansprüche, **dadurch gekennzeichnet, dass** die Polyurethandispersion eine anionische Dispersion auf der Basis von Polyacrylat, von Polyether, von Polyester oder von Polycarbonat und von Isocyanat, aliphatisch und/oder aromatisch, oder einem Gemisch derselben ist.

5. Zusammensetzung nach einem der vorangehenden Ansprüche, **dadurch gekennzeichnet, dass** die Polyurethandispersion Partikel in einer Größe unter 200 nm, vorzugsweise unter 100 nm einschließt und einen Trockenextraktwert aufweist, der zwischen 20 und 50 Gew.-%, vorzugsweise zwischen 20 und 40 Gew.-% liegt.

6. Zusammensetzung nach einem der vorangehenden Ansprüche, **dadurch gekennzeichnet, dass** die Polyurethandispersion eine Filmbildungstemperatur von unter oder gleich 30 °C, vorzugsweise von unter 20 °C aufweist.

7. Zusammensetzung nach einem der vorangehenden Ansprüche, **dadurch gekennzeichnet, dass** sie vorzugsweise bis 30 Gew.-% einer wässrigen Acrylpolymer- oder Acrylstyroldispersion, deren Partikel eine Größe unter 150 nm und vorzugsweise unter 100 nm aufweisen, als komplementären Filmbildner einschließt.

8. Zusammensetzung nach einem der vorangehenden Ansprüche, **dadurch gekennzeichnet, dass** sie ein oder mehrere Pigmente und/oder Zuschläge, organische oder anorganische, in einem Anteil von unter oder gleich 10 %, vorzugsweise von unter oder gleich 5 Gew.-%, in Bezug auf das Gesamtgewicht der Zusammensetzung einschließt.

9. Zusammensetzung nach einem der vorangehenden Ansprüche, **dadurch gekennzeichnet, dass** sie ebenfalls mindestens ein Additiv enthält, ausgewählt aus: Co-Lösungsmitteln, Koaleszenzen, Anti-Schaum-Mitteln, oberflächenaktiven Mitteln, Benetzungsmitteln, Dispersionsmitteln, Wachsen, Silikonen, Trocknungsbeschleunigern, Vernetzungsmitteln, Haftpromotern, UV-Filtern, Rheologiemitteln wie Acrylverdicker und assoziative Polyurethane, Konservierungsmitteln wie antibakterielle und Antipilzmittel, die für den Schutz des wässrigen Milieus notwendig sind, oder ein Gemisch derselben.

10. Zusammensetzung nach einem der vorangehenden Ansprüche, **dadurch gekennzeichnet, dass** sie ein oder mehrere aktive Mittel für die Pflege und die Schönheit der Nägel enthält, vorzugsweise mit nicht therapeutischer Wirkung.

11. Zusammensetzung nach einem der vorangehenden Ansprüche, **dadurch gekennzeichnet, dass** sie imstande ist, auf dem Nagel einen Film zu bilden, der nach dem Trocknen durch einfaches Abziehen abschminkbar ist.

12. Verfahren zum Schminken und Abschminken von Nägeln, **dadurch gekennzeichnet, dass** es die folgenden Schritte umfasst:
i) Auftragen von einer oder mehreren Schichten einer Lackzusammensetzung nach einem der Ansprüche 1 bis 11 in Form eines kontinuierlichen Films auf den Nagel,
ii) Trocknen jeder der Lackschichten bei Raumtemperatur,
iii) Abschminken des Lacks durch Abziehen des auf dem Nagel aufgebrachten Films.

## Claims

1. Aqueous, peel-off nail varnish composition containing as film-forming agent an aqueous polyurethane dispersion, **characterized in that** it further comprises from 2 to 5 weight % of an aqueous dispersion of anionic colloidal silica containing sodium or ammonium counter-ions and between 30 and 40 weight % of silica particles.

2. The composition according to claim 1, **characterized in that** the aqueous dispersion of colloidal silica contains particles of colloidal silica of size between 5 and 45 nm.

3. The composition according to one of claims 1 or 2, **characterized in that** the size of the colloidal silica particles is between 5 and 30 nm, more preferably between 7 and 25 nm.

4. The composition according to any of the preceding claims, **characterized in that** the polyurethane dispersion is an anionic dispersion of a polyacrylate, polyether, polyester or polycarbonate, and aliphatic and/or aromatic isocyanate, or a mixture thereof.

5. The composition according to any of the preceding claims, **characterized in that** the polyurethane dispersion contains particles of size less than 200 nm, preferably less than 100 nm, and has a dry extract value of between 20 and 50 weight %, preferably between 20 and 40 weight %.

6. The composition according to any of the preceding claims, **characterized in that** the polyurethane dispersion has a film-forming temperature lower than or equal to or 30 °C, preferably lower than 20 °C.

7. The composition according to any of the preceding claims, **characterized in that** it preferably contains up to 30 weight % of an aqueous dispersion of acrylic or styrene acrylic polymer having particles of size less than 150 nm and preferably less than 100 nm, as additional film-forming agent.

8. The composition according to any of the preceding claims, **characterized in that** it contains one or more organic or inorganic pigments and/or fillers in a proportion lower than or equal to 10 weight %, preferably lower than or equal to 5 weight % relative to the total weight of the composition.

9. The composition according to any of the preceding claims, **characterized in that** it further contains at least one additive selected from among: co-solvents, coalescing agents, defoaming agents, surfactants, wetting agents, dispersing agents, waxes, silicones, drying accelerators, crosslinking agents, adhesion promoters, UV filters, rheological agents such as acrylic and polyurethane associative thickeners, preserving agents such as antibacterial and antifungal agents required to preserve the aqueous medium, or a mixture thereof.

10. The composition according to any of the preceding claims, **characterized in that** it contains one or more active substances for nail care and beauty, preferably having non-therapeutic effect.

11. The composition according to any of the preceding claims, **characterized in that** it is able to form a film on a nail which, after drying, can be removed simply by peel-off.

12. Method for nail makeup application and removal, **characterized in that** it comprises the following steps:
i) applying to the nail one or more layers of a varnish composition according to any of claims 1 to 11, in the form of a continuous film;
ii) drying each layer of varnish at ambient temperature;
iii) removing the varnish by peeling off the film deposited on the nail.
